**Europäisches Patentamt**

**European Patent Office**

**Office européen des brevets**

(11) Veröffentlichungsnummer : **0 307 440 B1**

(12)
# EUROPÄISCHE PATENTSCHRIFT

(45) Veröffentlichungstag der Patentschrift :
**17.06.92 Patentblatt 92/25**

(51) Int. Cl.$^5$ : **G02B 7/10,** A61B 1/04

(21) Anmeldenummer : **88902807.2**

(22) Anmeldetag : **31.03.88**

(86) Internationale Anmeldenummer :
**PCT/DE88/00213**

(87) Internationale Veröffentlichungsnummer :
**WO 88/07694 06.10.88 Gazette 88/22**

(54) **OBJEKTIVFASSUNG FÜR EIN VARIOOBJEKTIV FÜR FERNSEHKAMERAS.**

(30) Priorität : **31.03.87 DE 3710646**

(43) Veröffentlichungstag der Anmeldung :
**22.03.89 Patentblatt 89/12**

(45) Bekanntmachung des Hinweises auf die
Patenterteilung :
**17.06.92 Patentblatt 92/25**

(84) Benannte Vertragsstaaten :
**AT BE CH DE FR GB IT LI LU NL SE**

(56) Entgegenhaltungen :
**FR-A-14 591 04**
**US-A- 4 639 772**
**US-A-17 608 27**

(56) Entgegenhaltungen :
**Research Disclosure, Nr. 116, Dezember 1973,
D.P. Davey et al.: "Zoom lens mount", siehe
Seiten 57-58
Patent Abstracts of Japan, Band 10, Nr. 165
(P-467)(2221), 12. Juni 1986 & JP, A, 6118910**

(73) Patentinhaber : **LEMKE, Norbert
Veilchenstrasse 10
W-8031 Puchheim (DE)**

(72) Erfinder : **LEMKE, Norbert
Veilchenstrasse 10
W-8031 Puchheim (DE)**

(74) Vertreter : **Münich, Wilhelm, Dr. et al
Kanzlei Münich, Steinmann, Schiller
Willibaldstrasse 36/38
W-8000 München 21 (DE)**

EP 0 307 440 B1

## Beschreibung

## Technisches Gebiet

Die Erfindung bezieht sich auf ein Varioobjektiv für eine Fernsehkamera gemäß dem Oberbegriff des Patentanspruchs 1.

## Stand der Technik

Beispielsweise bei der Endoskopie werden verstärkt Fernsehkameras für die Aufnahme des Endoskopie-Bildes eingesetzt. Um den behandelnden Arzt bei der Operation nicht zu behindern, müssen die Fernsehkameras möglichst klein und leicht sein. Deshalb sind bislang Fernsehkameras mit Varioobjektiven, die eine Einstellung des Gesichtsfelds und eine Anpassung an unterschiedliche Instrumente ermöglichen, nur vereinzelt in Betracht gezogen worden, da die bekannten Objektivfassungen für Varioobjektive vergleichsweise groß und schwer sind.

Aus der US-A-1 760 827 ist zwar bereits eine vergleichsweise kleine Objektivfassung bekannt; diese bekannte Objektivfassung ist aber für ein Objektiv mit fester Brennweite konzipiert und ermöglicht insbesondere nicht die bei einem Varioobjektiv erforderlichen unterschiedlichen Einstellvorgänge für die einzelnen Linsengruppen.

## Beschreibung der Erfindung

Der Erfindung liegt die Aufgabe zugrunde, ein Varioobjektiv für eine Fernsehkamera, die insbesondere an einem Endoskop angebracht werden soll, mit möglichst kleinen Abmessungen und geringem Gewicht anzugeben.

Eine erfindungsgemäße Lösung dieser Aufgabe ist mit ihren Weiterbildungen in den Patentansprüchen gekennzeichnet.

Erfindungsgemäß ist der Objektivtubus in einem Lagerbock, der in Bezug auf das Gehäuse der Fernsehkamera fixiert ist, in Richtung der optischen Achse des Objektivs verschiebbar geführt und gegen Drehbewegungen gesichert. Ferner sind die Führungsausnehmungen Schlitze, die am vorderen und hinteren Ende des Objektivtubus offen sind, und die aus einem geraden, parallel zur optischen Achse verlaufenden Teil im Anschluß an das vordere bzw. hintere Ende des Tubus, einem konzentrisch zur optischen Achse verlaufenden Teil und der sich an diesen Teil anschließenden eigentlichen Kurvenbahn zur Steuerung der Bewegung der Linsengruppen bestehen.

Hierdurch ergeben sich folgende Vorteile:

Das Einsetzen der Linsenfassungen in den Objektivtubus kann durch die vorne bzw. hinten offenen Schlitze erfolgen, so daß es nicht erforderlich ist, den Objektivtubus aus zwei Halbschalen zu fertigen, die dann durch den Durchmesser vergrößernde Mittel fest miteinander verbunden werden müssen. Auch der am Ende des Objektivtubus angeordnete Lagerbock, der eine Drehbewegung verhindert, andererseits jedoch eine Verschiebung zum Fokussieren des Objektivs erlaubt, erhöht nicht den Durchmesser des Varioobjektivs.

Durch die einteilige Konstruktion des Objektivtubus ist es ausreichend, auf dem Objektivtubus einen zweiten Tubus drehbar anzuordnen, in dessen Innenfläche zur optischen Achse parallele Ausnehmungen vorgesehen sind, in die die Zapfen eingreifen. Durch Drehen dieses zweiten Tubus kann dann leicht die Brennweite des Varioobjectivs eingestellt werden.

Weiterbildungen der Erfindung sind in den Unteransprüchen angegeben.

Selbstverständlich ist es möglich, zur Variation der Brennweite den zweiten Tubus von Hand oder mittels eines Einstellhebels zu drehen.

Besonders vorteilhaft ist es jedoch, wenn auf der Außenfläche des zweiten Tubus eine Verzahnung vorgesehen ist, die mit einem Kronenrad in Eingriff steht (Anspruch 2), da dann zum Einstellen der Brennweite ein einfaches Rändelrad auf der Außenseite des Varioobjektivs genügt, das - im Gegensatz zu einem zur optischen Achse konzentrischen Einstellring - eine einfache Einhandbedienung sowohl der Brennweite als auch der Fokussierung (Anspruch 5) ermöglicht. Das Rändelrad zur Fokussierung kann dabei beispielsweise über Zapfen in eine exzentrische elliptische Ausnehmung im zweiten Tubus eingreifen.

Durch die im Anspruch 3 gekennzeichnete Weiterbildung wird die Stabilität des geschlitzten Objektivtubus und die Stabilität der Einstellung wesentlich erhöht, da die Linsenfassungen einerseits den Objektivtubus stabilisieren und andererseits durch diese Ausbildung nicht verkippen können.

Die im Anspruch 4 gekennzeichnete Ausbildung, bei der zwei sich parallel zur optischen Achse erstreckende Ansätze vorgesehen sind, ermöglicht einen sehr kompakten Aufbau, da der Lagerbock an der Trennstelle zwischen Objektiv und Röhre der Fernsehkamera, an der er praktisch keinen zusätzlichen Platz benötigt, vorgesehen werden kann.

Die im Anspruch 5 gekennzeichnete Ausbildung, bei der sowohl zum Fokussieren als auch zur Brennweitenverstellung Rändelräder vorgesehen sind, ist ergonomisch günstig, da eine Einhandbedienung ohne Änderung der Griffstellung möglich wird.

Weiterhin ist es leicht möglich, eine "Wasserkante" vorzusehen, durch die gerade beim Anflanschen des erfindungsgemäßen, Varioobjektivs an ein Endoskop keine weiteren, das Volumen und das Gewicht erhöhenden Maßnahmen, wie sie nach dem Stand der Technik erforderlich sind, getroffen werden müssen, um zu verhindern, daß Wasser auf die vordere Abdeckung läuft.

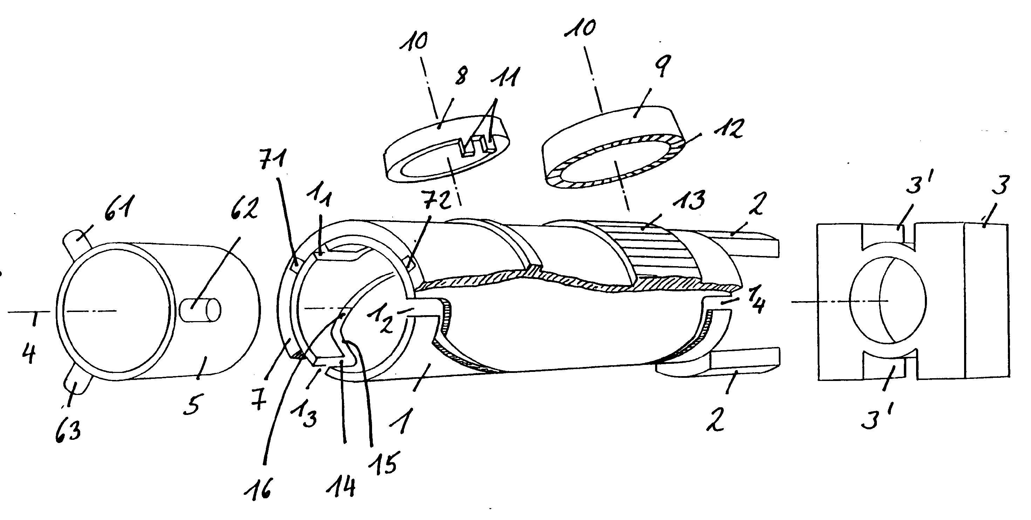

Die Erfindung wird nachstehend anhand eines Ausführungsbeispiels unter Bezugnahme auf die Zeichnung näher beschrieben, deren einzige Figur perspektivisch und auseinandergezogen die Fassung eines erfindungsgemäßen Varioobjektivs zeigt.

Das erfindungsgemäße Objektiv weist einen Objektivtubus 1 mit zwei Ansätzen 2 auf, mittels derer er in einem Lagerbock 3 mittels Ausnehmung oder Schlitzen 3′ in Richtung der optischen Achse 4 verschiebbar geführt ist.

In dem Objektivtubus 1 sind zwei Linsengruppen zum Einstellen der Brennweite des Objektivs längsverschiebbar geführt. Hierzu weisen die Linsengruppen Linsenfassungen 5 auf, von denen in der Figur nur die vordere dargestellt ist. Jede der Linsenfassungen 5 hat drei radial verlaufende Zapfen 61 bis 63, die als Schlitze $1_1$ bis $1_6$ ausgebildete Steuerkurven im Objektivtubus 1 durchsetzen und in gerade Nuten 71, 72,... in einem zweiten Tubus 7 eingreifen, der auf dem Objektivtubus 1 drehbar angeordnet ist.

Ferner sind zwei drehbare Räder 8 und 9, deren Drehachse 10 senkrecht auf der optischen Achse 4 des Objektivs steht, zum Einstellen der Fokussierung und der Brennweite vorgesehen.

Das zum Einstellen der Fokussierung dienende Rad 8 weist Zapfen 11 auf, die in eine exzentrische elliptische Ausnehmung im zweiten Tubus 7 eingreifen. Damit kann durch Drehen des Rades 8 das im Lagerbock 3 verschiebbar geführte Varioobjektiv längs seiner optischen Achse 4 verschoben werden.

Auf der Unterseite des zweiten Rades 9 ist eine Kronenverzahnung 11 vorgesehen, die mit einer auf dem zweiten Tubus 7 vorgesehenen Verzahnung 13 kämmt. Damit kann durch Drehen des zweiten Rades der zweite Tubus auf dem Objektivtubus gedreht werden, so daß sich die Linsengruppen entsprechend den Steuerkurven zur Einstellung der Brennweite bewegen.

Die Schlitze $1_1$ bis $1_6$, die als Steuerkurven für die Linsengruppen dienen, bestehen aus drei Abschnitten: Einem parallel zur optischen Achse 4 verlaufenden Abschnitt 14, der ein Einsetzen der Linsenfassung ermöglicht, einem Abschnitt 15, der konzentrisch zur optischen Achse verläuft und verhindert, daß die Linsenfassung aus der Steuerkurve durch Welterdrehen des zweiten Tubus 7 herausgedreht werden kann, und der eigentlichen Steuerkurve 16, die die Bewegung der Linsengruppe bei der Brennweiteneinstellung steuert.

Vorstehend ist die Erfindung anhand eines Ausführungsbeispiels ohne Beschränkung des allgemeinen Erfindungsgedankens - ein Objektiv zu schaffen, bei dem die Linsenfassung in den Steuertubus eingesetzt werden können, ohne diesen aus zwei Halbschalen zusammenzusetzen - beschrieben worden. Innerhalb dieses allgemeinen Erfindungsgedankens sind selbstverständlich die verschiedensten Modifikationen möglich:

Beispielsweise kann die Brennweitenverstellung auch durch direktes Verdrehen des zweiten Tubus erfolgen.

Ferner ist es möglich, die erfindungsgemäße Linsenfassung für das Varioobjektiv direkt und nicht abnehmbar mit der Fernsehkamera oder abnehmbar über Bajonett- oder C-Mount-verbindungen zu koppeln.

Weiterhin ist die Ausbildung der Linsen der einzelnen Linsengruppen weitgehend frei wählbar:

Das erfindungsgemäß ausgebildete Varioobjektiv ist durch den besonderen Aufbau der Linsenfassung bereits sehr kompakt. Eine weitere Reduzierung des Gewichts und der bemessungen wird dadurch erreicht, daß jede der Linsengruppen lediglich aus einem Kittglied besteht. Erfindungsgemäß ist erkannt worden, daß ein derartiger Aufbau bereits für eine Fernsehkamera zur Abbildung eines Endoskopie-Bildes ausreichend ist.

Ferner kann bei Benutzung des erfindungsgemäßen Varioobjektivs in Verbindung mit einem Endoskop eine Wasserkante an der vorderen Abdeckung vorgesehen werden, die verhindert, daß Wasser vor die Frontabdeckung läuft und beispielsweise Spülluftanschlüsse überflüssig macht.


## Patentansprüche

1. Varioobjektiv für eine Fernsehkamera, die insbesondere an einem Endoskop anbringbar ist, mit zwei Linsengruppen, die jeweils in einer Linsenfassung (5) gehalten sind, und die zum Verstellen der Brennweite gegeneinander und zum Fokussieren gemeinsam verschiebbar sind, und einem Objektivtubus (1), der für jede Linsenfassung wenigstens eine Führungsausnehmung ($1_1$,..$1_6$) aufweist,

in die an den Linsenfassungen (5) angebrachte radiale Zapfen (61..63) eingreifen,
**gekennzeichnet** durch die Kombination folgende Merkmale:

– der Objektivtubus (1) ist in einem Lagerbock (3) in Richtung der optischen Achse (4) des Objektivs verschiebbar geführt und ist gegen Drehbewegungen gesichert,

– die Führungsausnehmungen sind Schlitze ($1_1$,..$1_6$), die am vorderen bzw. hinteren Ende des Objektivtubus (1) offen sind und die aus einem geraden, parallel zur optischen Achse verlaufenden Teil (14) im Anschluß an das vordere bzw. hintere Ende des Objektivtubus, einem konzentrisch zur optischen Achse verlaufenden Teil (15) und der sich an diesen Teil anschließenden eigentlichen Kurvenbahn (16) zur Steuerung der Bewegung der Linsengruppen bestehen,

– auf dem Objektivtubus ist ein zweiter Tubus (7) drehbar angeordnet, dessen Innenfläche zur optischen Achse parallele Ausnehmungen (71,..) aufweist,in die die Zapfen (61,..) eingreifen.

2. Varioobjektiv nach Anspruch 1,
dadurch **gekennzeichnet**, daß auf der Außenfläche des zweiten Tubus eine Verzahnung (13) vorgesehen ist, die mit einem Kronenrad (12) in Eingriff steht, das zum Einstellen der Brennweite dient.

3. Varioobjektiv nach Anspruch 1 oder 2,
dadurch **gekennzeichnet**, daß jede Linsenfassung (5) drei um 120° versetzte Zapfen (61,..63) aufweist, die in entsprechende Führungsschlitze und Ausnehmungen auf der Innenseite des zweiten Tubus eingreifen.

4. Varioobjektiv nach Anspruch 1 bis 3,
dadurch **gekennzeichnet**, daß der Objektivtubus zwei sich parallel zur optischen Achse erstreckende Ansätze (2) aufweist, die in komplementäre Schlitze bzw. Ausnehmungen (3') in dem Lagerbock eingesetzt sind.

5. Varioobjektiv nach einem der Ansprüche 1 bis 4,
dadurch **gekennzeichnet**, daß jeweils ein Rändelrad (8,9), dessen Drehachse (10) senkrecht zur optischen Achse (4) steht, zum Fokussieren und zur Brennweitenverstellung vorgesehen ist.

## Claims

1. Varifocal lens for a TV camera, which is adapted in particular for mounting on an endoscope, comprising: two lens groups which are each mounted in a lens mount (5) and adapted to be shifted relative to one another for setting the focal length and to be shifted together to focus the lens, and a lens barrel (1) which has at least one guide indentation ($1_1$, ... $1_6$) for each lens mount for engagement of radial pins (61 ... 63) provided at said lens mounts (5),
**characterized** by the combination of the following features:

– the lens barrel (1) is guided in a support bearing (3) for displacement in the direction of the optical axis (4) of said lens and is secured against rotary motion,

– the guiding indentations are slits ($1_1$, ... $1_6$) which are open at the front or rear end, respectively of said lens barrel and which consist of a straight section (14) parallel to the optical axis, that joins the front or rear end, respectively of said lens barrel, of a section (15) extending concentrically to the optical axis, and the cam (16) proper joining that latter section for controlling the movement of said lens groups,

– a second barrel (7) is mounted for rotation on said lens barrel and has an inner surface provided with indentations parallel to the optical axis for engagement of said pins (61, ...).

2. Varifocal lens according to Claim 1,
**characterized** in that a toothing (13) is provided on the outer surface of said second barrel, which meshes with a castellated wheel (12) that serves for setting the focal length.

3. Varifocal lens according to Claim 1 or 2,
**characterized** in that each lens mount (5) is provided with three pins (61, ... 63) spaced by 120° apart, which engage with corresponding guide slits and indentations on the inner surface of said second barrel.

4. Varifocal lens according to Claims 1 to 3,
**characterized** in that said lens barrel is provided with two lugs (2) extending parallel to the optical axis, which are inserted into complementary slits or indentations (3') in said support bearing.

5. Varifocal lens according to any of Claims 1 to 4,
**characterized** in that one knurled wheel (3, 9) each is provided for focusing and resetting the focal length, said knurled wheel having an axis of rotation (10) normal to the optical axis (4).

## Revendications

1. Objectif à focale variable pour un caméra de télévision, qui est apte en particulier pour le montage sur un endoscope, comprenant:

deux groupes d'objectifs dont chacun est monté dans la monture d'objectif (5) et pourvu à être déplacé relativement à l'autre groupe pour ajuster la distance focale, et qui sont pourvus tous les deux pour être déplacés en commun pour focaliser l'objectif,

ainsi qu'un tube d'objectif (1) à au moins un creux de guidage ($1_1$, ... $1_6$) pour chaque la monture d'objectif pour l' enagement des tourillons radiaux (61 ... 63) disposés auxdites montures d'objectif (5),

**caractérisé** par la combinaison des caractéristiques suivantes:

– le tube d'objectif (1) est guidé dans un support (3) pour un déplacement au sens de l'axe optique (4) dudit objectif et est arrêté contre une rotation,

– les creux de guidage sont des are coupures ($1_1$, ... $1_6$) ouvertes à l'extrémité avant ou respectivement arrière dudit tube d'objectif, qui consistent en un élément rectiligne (14) parallèle à l'axe optique, ce segment se joignant à l'extrémité avant ou respectivement arrière dudit tube d'objectif, en un élément (15) qui s'étend concentriquement à l'axe optique, et la came de commande (16) propres qui est jointe au dernier élément afin de contrôler le mouvement desdits groupes d'objectifs,

– un deuxième tube (7) est monté à tourner autour ledit tube d'objectif et présente une surface intérieure pourvue des creux parallèles à l'axe optique pour l'engagement desdits tourillons (61, ...).

2. Objectif à focale variable selon la revendication 1,

**caractérisé** en ce qu'une denture (13) est formée sur la surface extérieure dudit deuxième tube, qui s' engrène dans une roue à créneaux (12) qui sert à l'ajustage de la distance focale.

3. Objectif à focale variable selon la revendication 1 ou 2,

**caractérisé** en ce que chaque monture d'objectif (5) est pourvue de trois tourillons (61, ... 63) espacés l'un de l'autre par 120°, qui sont engagés dans des coupures de guidage et creux correspondants sur la surface intérieure dudit deuxième tube.

4. Objectif à focale variable selon les revendications 1 à 3,

**caractérisé** en ce que ledit tube d'objectif est pourvu de deux talons (2) qui s'étendent en parallèle à l'axe optique, lesquels talons sont insérés dans des coupures ou creux (3') complémentaires dans ledit support.

5. Objectif à focale variable selon une quelconque des revendications 1 à 4,

**caractérisé** en ce que respectivement une molette (3, 9) à axe de rotation (10) perpendiculaire à l'axe optique (4) est montée pour la focalisation et la variation de la distance focale.